# EUROPEAN PATENT APPLICATION

(11) **EP 3 248 970 A1**
(43) Date of publication of application: **29.11.2017**
(21) Application number: 17020225.3
(22) Date of filing: 23.05.2017
(51) Int. Cl.: C07D 453/02

(54) **FORMS OF UMECLIDINIUM BROMIDE**

(30) Priority: 27.05.2016 CZ 201632417 U; 27.05.2016 CZ 201633011 U; 27.05.2016 CZ 201633012 U
(71) Applicant: Zentiva K.S., 102 37 Praha 10 (CZ)
(72) Inventor: Cerna, Igor, 169 00 Praha 6 (CZ); Dammer, Ondrej, 253 01 Hostivice (CZ); Krejcik, Lukas, 190 17 Praha 9 (CZ)
(74) Representative: Jirotkova, Ivana

(57) **Abstract**

Solid forms of umeclidinium bromide, in particular its non-solvated form 1, novel crystalline forms A and B and an amorphous form, their characterization with the use of analytic methods, methods of their preparation and their use for the preparation of highly pure umeclidinium bromide.

## Description

### Technical Field

The invention relates to novel solid forms of umeclidinium bromide (I), chemically 1-[2-(benzyloxy)ethyl]-4-(hydroxydiphenylmethyl)-1-azabicyclo[2.2.2]octane bromide. In particular, to its novel crystalline forms, identified as form A and form B, as well as to an amorphous form, and to their characterization by means of analytic methods. The invention further relates to methods of their preparation and their use for the preparation of umeclidinium bromide in the API quality.

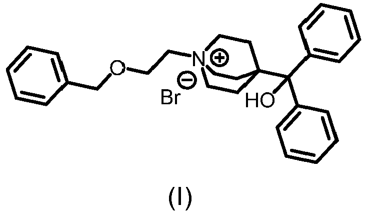

Umeclidinium bromide is indicated as an inhalation anticholinergic drug with an ultra-long-term effect in cooperating patients with the diagnosis of COPD (chronic obstructive pulmonary disease). COPD is defined as a preventable and treatable disease that is characterized by a persistent obstruction of air flow in the bronchi (bronchial obstruction), which usually progresses and is related to an intensified inflammatory response of the airways to harmful particles or gases. The main goal of the treatment of COPD is an improvement of the current control, i.e. elimination of symptoms, improvement of toleration of physical effort, improvement of the health condition and reduction of future risks, i.e. prevention and treatment of exacerbations, prevention of progression of the disease and mortality reduction.

### Background Art

The structure of umeclidinium bromide, 1-[2-(benzyloxy)ethyl]-4-(hydroxydiphenylmethyl)-1-azabicyklo[2.2.2]octane bromide, is first mentioned in the general patent application WO2005009362 of 2003.

Preparation of umeclidinium bromide is first disclosed in the patent EP 1 740 177B (WO2005104745), where two methods (A and B) are mentioned, differing in the final processing and the product yield (method B included in Scheme 1). There, the last steps of the synthesis are described, the product being described by means of EI-MS, ¹H NMR and elementary analysis. There is no information concerning the chemical purity or polymorphic form.

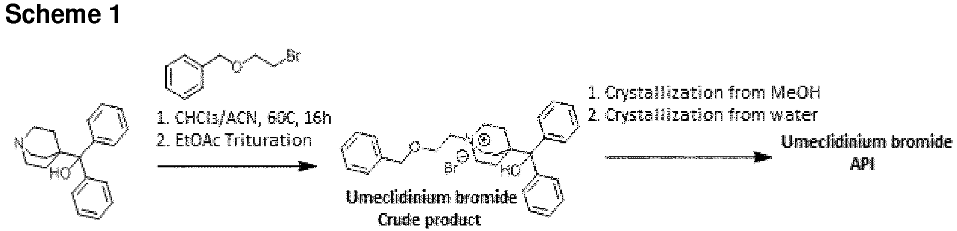

Another preparation method of umeclidinium bromide is disclosed in the patent application WO 2014027045, where three forms are also described (identified as forms 1 to 3), prepared using a method that is different from the procedure disclosed in the patent EP 1 740 177B.

### Summary of the Invention

The invention relates to solid forms of umeclidinium bromide, in particular the non-solvated anhydrous form 1, novel crystalline forms A and B and an amorphous form, their characterization with the use of analytic methods, methods of their preparation and their use for the preparation of highly pure umeclidinium bromide.

It has been found out that reproduction of the procedure of method B from Example 2 of the patent EP 1 740177B provided the non-solvated anhydrous form of umeclidinium bromide known as form 1 from the patent application WO 2014027045, characterized by the following characteristic reflections in the X-ray powder pattern: 6.7; 13.4; 20.1 and 24,3 ± 0.2° 2-theta, which were measured with the use of CuKα radiation. This form also shows more reflections: 11.5; 14.6; 22.4 and 26.9 ± 0.2° 2-theta. The chemical purity of the sample obtained this way achieved 99.8% according to UPLC. The melting point of form 1 obtained this way is 237°C as determined by DSC.

Form A of umeclidinium bromide was obtained by crystallization from other solvents of general formula ROH, where R can be a C1 to C4 carbon chain, and crystallization from these solvents in a mixture with water. A mixture of methanol and water is preferably used. The novel form A is characterized by these characteristic reflections in the X-ray powder pattern: 5.4; 10.9; 19.6 and 23,8 ± 0.2° 2-theta, which were measured with the use of CuKα radiation. This form also shows more reflections: 9.5; 16.3; 17.7; 21.6 and 27.5 ± 0.2° 2-theta. The chemical purity of the sample obtained this way achieved 99.9% according to UPLC. The melting point determined by means of DSC is 167.6°C.

Form B of umeclidinium bromide was obtained by loading of a sample of form A with a temperature higher than 180°C. The novel form B is characterized by these characteristic reflections in the X-ray powder pattern: 6.7; 18.3; 20.6 and 24,9 ± 0.2° 2-theta, which were measured with the use of CuKα radiation. This form also shows more reflections: 14.6; 15.9; 19.2, 23.0 and 23.8 ± 0.2° 2-theta. The melting point determined by means of DSC is 232°C.

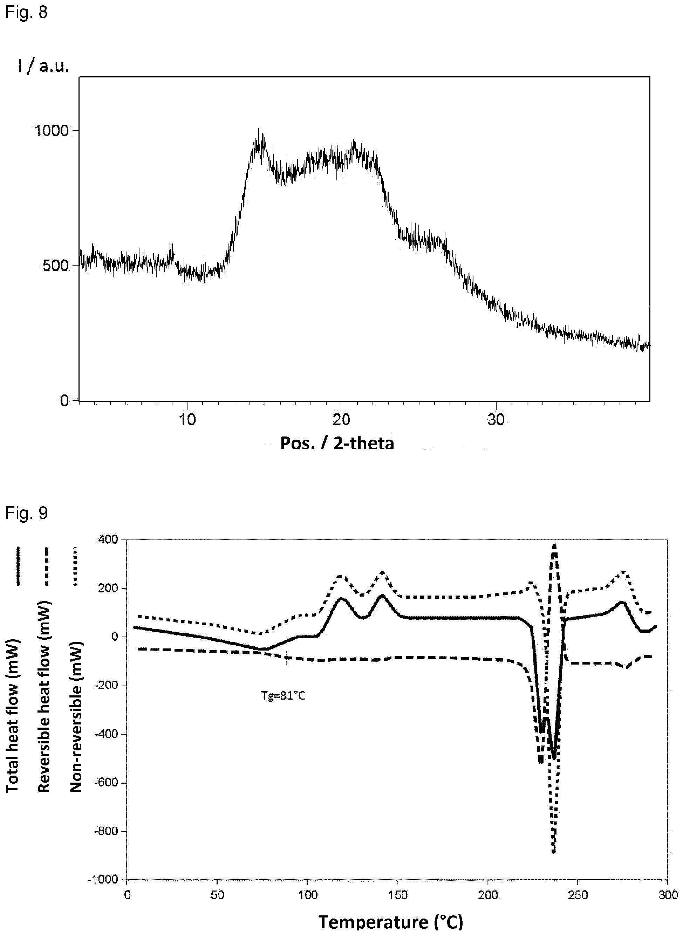

Lyophilization of a freeze-dried solution of umeclidinium bromide provided a so far not described amorphous form, characterized by an X-ray powder pattern characteristic for an amorphous form (included in the Appendix, Figure 8) and the glass transition temperature of 81 °C as determined by DSC (record in Figure 9).

### Detailed Description of the Invention

The invention relates to novel solid forms of umeclidinium bromide, in particular its non-solvated form 1, prepared using the procedure of the patent EP 1 740 177B, novel crystalline forms A and B and an amorphous form, their characterization with the use of analytic methods, methods of their preparation and their use for the preparation of highly pure umeclidinium bromide.

Form 1 of umeclidinium bromide is characterized by the powder XRPD pattern (Fig. 1) presented in Table 1, melting point of 237°C determined by the DSC thermal method (Fig. 2) and a TGA record (Fig. 3).

**Tab. 1: XRPD - diffraction peaks corresponding to the non-solvated anhydrous form 1 of umeclidinium bromide**

| Pos. [°2θ] | d [A = 0.1 nm] | Rel. Int. [%] |
|---|---|---|
| 6.70 | 13.190 | 100.0 |
| 8.90 | 9.932 | 2.0 |
| 10.02 | 8.819 | 2.0 |

| Pos. [°2θ] | d [A = 0.1nm] | Rel. Int. [%] |
|---|---|---|
| 11.45 | 7.719 | 5.6 |
| 13.39 | 6.607 | 15.5 |
| 13.70 | 6.457 | 8.8 |
| 14.55 | 6.082 | 5.1 |
| 15.29 | 5.789 | 4.3 |
| 17.31 | 5.119 | 2.6 |
| 17.76 | 4.990 | 3.7 |
| 18.66 | 4.752 | 2.5 |
| 19.84 | 4.472 | 13.4 |
| 20.14 | 4.406 | 16.3 |
| 22.39 | 3.968 | 6.1 |
| 23.03 | 3.860 | 5.3 |
| 23.60 | 3.767 | 2.0 |
| 24.26 | 3.666 | 6.4 |
| 25.40 | 3.504 | 1.4 |
| 25.91 | 3.436 | 2.5 |
| 26.94 | 3.307 | 5.0 |
| 27.64 | 3.225 | 4.1 |
| 28.32 | 3.149 | 4.1 |
| 29.25 | 3.051 | 1.0 |
| 29.57 | 3.018 | 0.8 |
| 29.97 | 2.979 | 0.7 |
| 30.77 | 2.903 | 3.9 |
| 31.84 | 2.809 | 1.6 |
| 33.33 | 2.686 | 0.8 |
| 33.84 | 2.646 | 0.9 |
| 34.62 | 2.589 | 2.4 |
| 35.06 | 2.558 | 1.5 |
| 37.03 | 2.426 | 1.1 |
| 38.11 | 2.360 | 1.7 |

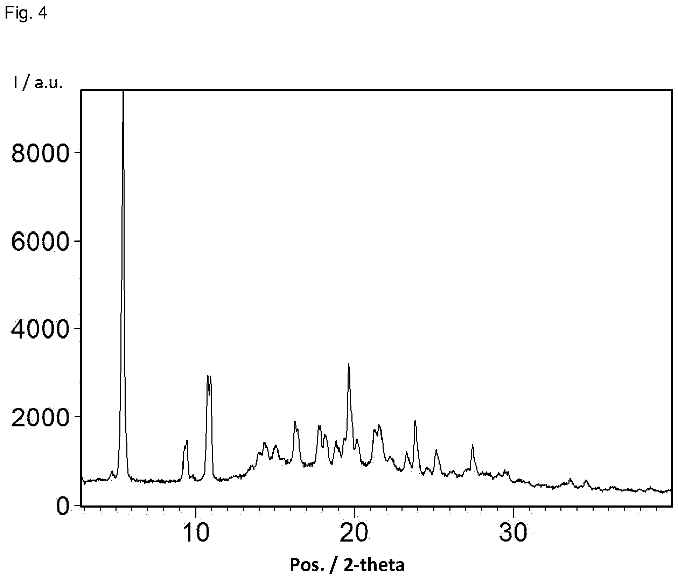

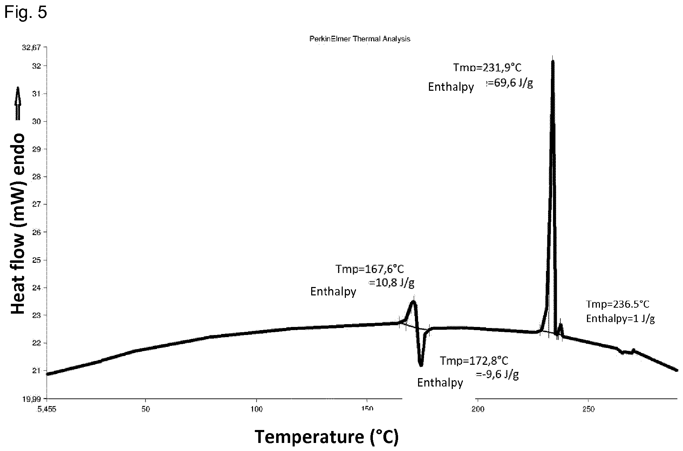

Form A is characterized by the powder XRPD pattern (Fig. 4) presented in Table 2 and the melting point of 167.6°C determined by the DSC thermal method (Fig. 5).

**Tab. 2: XRPD - diffraction peaks corresponding to form A of umeclidinium bromide**

| Pos. [°2θ] | d [A = 0.1 njm] | Rel. Int. [%] |
|---|---|---|
| 5.45 | 16.209 | 100.0 |
| 9.47 | 9.333 | 10.0 |
| 10.94 | 8.079 | 25.5 |
| 13.92 | 6.355 | 3.6 |
| 14.30 | 6.187 | 5.8 |
| 15.08 | 5.869 | 4.2 |
| 16.25 | 5.449 | 10.8 |
| 17.71 | 5.005 | 8.5 |
| 18.10 | 4.898 | 6.9 |
| 18.83 | 4.709 | 5.3 |
| 19.60 | 4.525 | 25.1 |
| 20.11 | 4.411 | 6.1 |
| 21.19 | 4.189 | 8.9 |
| 21.55 | 4.121 | 10.0 |
| 22.27 | 3.988 | 2.5 |
| 23.23 | 3.827 | 4.5 |
| 23.78 | 3.738 | 13.0 |
| 24.49 | 3.631 | 1.3 |
| 25.08 | 3.547 | 5.1 |
| 27.46 | 3.246 | 7.0 |
| 29.04 | 3.073 | 1.4 |
| 29.39 | 3.036 | 2.5 |
| 33.54 | 2.670 | 1.8 |
| 34.49 | 2.598 | 1.7 |

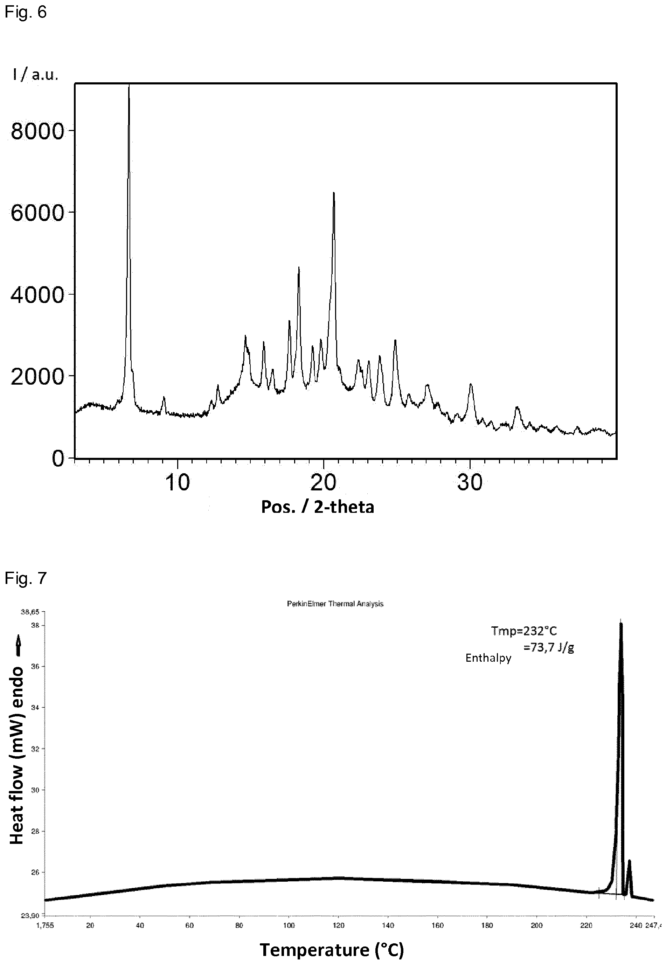

Form B is characterized by the powder XRPD pattern (Fig. 6) presented in Table 2 and the melting point of 232°C determined by the DSC thermal method (Fig. 7).

**Tab. 3: XRPD - diffraction peaks corresponding to form B of umeclidinium bromide**

| Pos. [°2θ] | d [Å = 0.1 nm] | Rel.Int. [%] |
|---|---|---|
| 6.66 | 13.258 | 100.0 |
| 9.08 | 9.732 | 6.4 |
| 12.33 | 7.174 | 2.9 |
| 12.77 | 6.926 | 4.9 |
| 14.61 | 6.059 | 9.6 |
| 15.91 | 5.567 | 12.3 |
| 16.48 | 5.374 | 5.5 |
| 17.62 | 5.030 | 17.6 |
| 18.27 | 4.853 | 31.6 |
| 19.19 | 4.621 | 10.3 |
| 19.79 | 4.482 | 12.0 |
| 20.60 | 4.308 | 45.9 |
| 22.32 | 3.980 | 7.3 |
| 23.04 | 3.856 | 9.3 |
| 23.79 | 3.737 | 11.3 |
| 24.87 | 3.578 | 16.5 |
| 25.81 | 3.448 | 3.2 |
| 27.00 | 3.299 | 8.4 |
| 30.04 | 2.972 | 13.6 |
| 33.23 | 2.694 | 6.5 |

The amorphous form of umeclidinium bromide is characterized by a powder XRPD pattern (Fig. 8) and a DSC record with the glass transition temperature of 81 °C (Fig. 9).

### Preparation method of the said forms of umeclidinium bromide.

Form 1 was prepared by reproduction of procedure B from Example 2 of the patent EP1740177B. In a preferred embodiment, umeclidinium bromide is dissolved, or suspended at an elevated temperature (60 to 70°C) in a suitable amount of water (12 ml/g of the API) and at this temperature it is left to be stirred for 1 to 2 hours. Subsequently, the mixture is left to cool down to the room temperature (20 to 25°C).

The produced suspension is filtered and washed with water. The isolated substance is dried at an elevated temperature (30 to 50°C) in vacuum (10,000 to 30,000 Pa) for at least 16 h.

Form A is produced by crystallization from a solution of umeclidinium bromide in a mixture of methanol and water in the volume ratio of 1:1 to 2:1. In a preferred embodiment, the substance is dissolved in methanol under boiling (concentration 15 to 25 ml of MeOH per 1 g of the API), subsequently, a suitable amount of water (7 to 25 ml per 1 g of the API) is added at an elevated temperature (approx. 60°C) and the solution is left to be stirred under boiling for 1 to 2 hours with the possibility of evaporation of a certain amount of the solvent (20 to 50%). Subsequently, the mixture is left to cool down to the room temperature (20 to 25°C). The thick suspension is then diluted with water, filtered and washed with water. The isolated substance is dried at an elevated temperature (30 to 50°C) in vacuum (10,000 to 30,000 Pa) for at least 24 h.

Form B of umeclidinium bromide was obtained by loading of a sample of form A with a temperature higher than 180°C.

The amorphous form of umeclidium bromide is obtained by lyophilization of a freeze-dried solution of the substance in a mixture of the solvents water and *tert-*butanol in the volume ratio of 6:4. The freeze-dried solution is subjected to high vacuum for one to three days.

### Brief Description of the Drawings

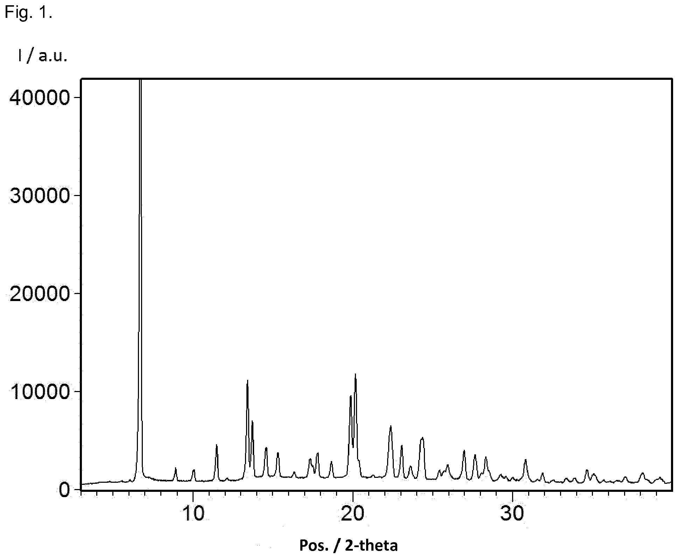

Fig. 1. XRPD pattern of form 1 of umeclidinium bromide

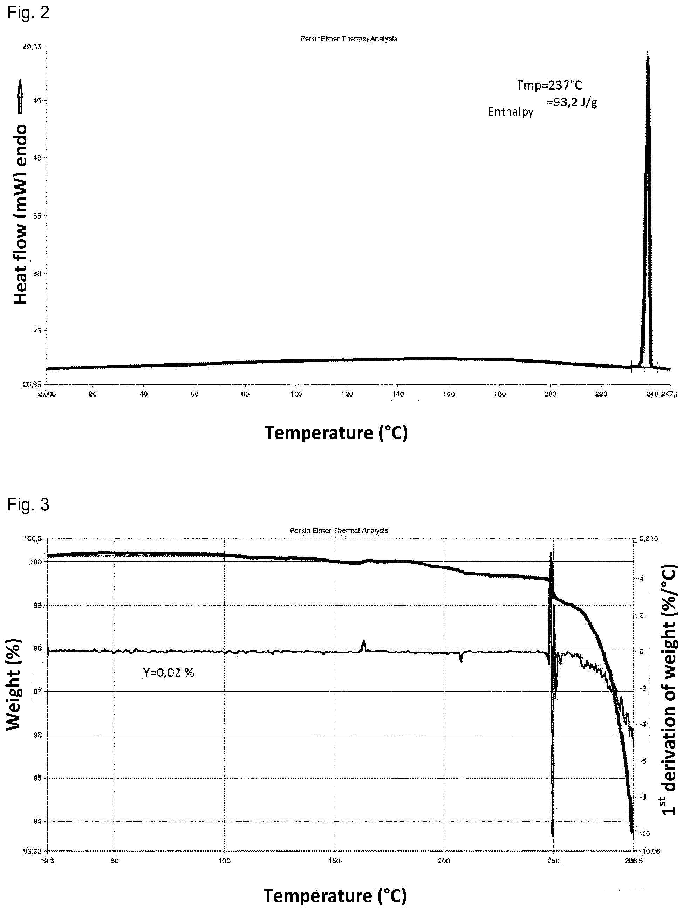

Fig. 2 DSC record of form 1 of umeclidinium bromide
Fig. 3 TGA record of form 1 of umeclidinium bromide
Fig. 4 XRPD pattern of form A of umeclidinium bromide
Fig. 5 DSC record of form A of umeclidinium bromide
Fig. 6 XRPD pattern of form B of umeclidinium bromide
Fig. 7 DSC record of form B of umeclidinium bromide
Fig. 8 XRPD pattern of the amorphous form of umeclidinium bromide
Fig. 9 DSC record of the amorphous form of umeclidinium bromide

### Examples

### List of analytical methods

**Measurement parameters of XRPD:** The diffractograms were measured using an X'PERT PRO MPD PANalytical diffractometer, used radiation CuKa (λ=1.542 A), excitation voltage: 45 kV, anode current: 40 mA, measured range: 2 - 40° 2θ, increment: 0.-01° 2θ, the measurement was carried out on a flat powder sample that was applied on a Si plate and covered with Kapton foil. For the setting of the primary optical equipment programmable divergence slits with the irradiated area of the sample of 10 mm, 0.02 rad Soller slits and a ¼° anti-diffusion slit were used. For the setting of the secondary optical equipment an X'Celerator detector with maximum opening of the detection slot, 0.02 rad Soller slits and a 5.0 mm anti-diffusion slit were used.

**The records of the differential scanning calorimetry (DSC)** *a) for the crystalline form of umeclidinium bromide* were measured using a DSC Pyris 1 device made by the company Perkin Elmer. The sample charge in a standard Al pot was between 3 mg and the heating rate was 10°C/min. The temperature program that was used consists of 1 min of stabilization at the temperature of 0°C and then of heating up to 250°C at the heating rate of 10°C/min. As the carrier gas 4.0 N₂ was used at the flow of 20 ml/min.
*b) for the amorphous form of umeclidinium bromide* were measured with the Discovery DSC device made by TA Instruments. The sample charge in a standard Al pot (40 µL) was 4-5 mg and the heating rate was 5°C/min. The temperature program that was used consists of 5 min of stabilization at the temperature of 0°C and then of heating up to 300°C at the heating rate of 5°C/min (Amplitude = 0.8°C and Period = 60 s). As the carrier gas 5.0 N₂ was used at the flow of 50 ml/min.

**The records of the thermogravimetric analysis (TGA)** were measured using a TGA 6 device made by the company Perkin Elmer. The sample charge in a corundum pot was about 20 mg and the heating rate was 10°C/min. The temperature program that was used consists of 1 minute's stabilization at the temperature of 15°C and then of heating up to 300°C at the heating rate of 10°C/min. As the carrier gas 4.0 N₂ was used at the flow of 20 ml/min.

**The liquid chromatography records (HPLC)** were measured with an Acquity device made by Waters with a PDA detector.
Measurement parameters of HPLC:

| | |
|---|---|
| mobile phase: | A: 2 ml of perchloric acid 70% are dissolved in 1 l of MQ water. |
| | B: acetonitrile |
| elution: | gradient |

| Time (min.) | Flow (ml/min.) | % A | % B |
|---|---|---|---|
| 0 | 0.8 | 75 | 25 |
| 1 | 0.8 | 75 | 25 |
| 9 | 0.8 | 30 | 70 |
| 10 | 0.8 | 30 | 70 |
| 11 | 0.8 | 75 | 25 |
| 12 | 0.8 | 75 | 25 |

| | |
|---|---|
| column: | XSelect CSH Phenyl-Hexyl 2.5 µm, 4.6 x 100 mm |
| sample solvent: | Methanol : Water (50:50, vol./vol.) |
| detection: | spectrophotometric 211 nm |
| injected amount: | 1 µl |
| autosampler temperature: | 18°C |
| column temperature: | 35°C |
| analysis time: | 12 min |
| sample concentration: | 1 mg/ml |

### Example 1

### Preparation of form 1 of umeclidinium bromide

1-[2-(Benzyloxy)ethyl]-4-(hydroxydiphenylmethyl)-1-azabicyclo[2.2.2]octane bromide (66.4 g, 0.13 mol, UPLC purity 99.67%) is suspended in 800 ml of water, the mixture is heated up to 55°C and stirred for 55 minutes. Subsequently, it is left to cool down to 20°C and filtered through frit. The filtered product is washed with 150 ml of water and dried in a vacuum drier at 45°C for 16 hours. The amount of 61.4 g of umeclidinium bromide of form 1 was obtained in the yield of 92% with the purity of 99.78%. The X-ray powder diffraction pattern is shown in the Annex in Fig. 1, DSC record in Fig. 2 and TGA record in Fig. 3.

### Example 2

### Preparation of form 1 of umeclidinium bromide

1-[2-(Benzyloxy)ethyl]-4-(hydroxydiphenylmethyl)-1-azabicyclo[2.2.2]octane bromide (66.6 g, 0.13 mol, UPLC purity 99.67%) is suspended in 800 ml of water, the mixture is heated up to 65°C and stirred for 1.5 hours. Subsequently, the weak suspension is left to cool down to 20°C and filtered through frit. The filtered product is washed with 150 ml of water and dried in a vacuum drier at 45°C for 18 hours. The amount of 55.7 g of umeclidinium bromide of form 1 was obtained in the yield of 84% with the purity of 99.81%. The X-ray powder diffraction pattern was equal to the X-ray powder diffraction pattern of Example 1.

### Example 3

### Preparation of form A of umeclidinium bromide

1-[2-(Benzyloxy)ethyl]-4-(hydroxydiphenylmethyl)-1-azabicyclo[2.2.2]octane bromide (327 mg, 0.643 mmol, UPLC purity 98.89%) is dissolved in 8 ml of methanol at 60°C and 4.3 ml of water are slowly added by dripping to the stirred solution and the solution is left to be refluxed for 1.5 hours. Then, the mixture is cooled down to the room temperature (20 to 25°C). The obtained thick suspension is diluted with water (3 to 5 ml) and filtered through frit. The filtered product is washed with 5 ml of water and dried in a vacuum drier at 45°C for 24 hours, 13,600 Pa. The amount of 188.7 g of umeclidinium bromide of form A was obtained in the yield of 70% with the purity of 99.96%. The X-ray powder diffraction pattern is shown in Fig. 4 and the DSC record in Fig. 5.

### Example 4

### Preparation of form B of umeclidinium bromide

Form A (50 mg) was dried at the atmospheric pressure at 200°C. The X-ray powder diffraction pattern is shown in Fig. 6 and the DSC record in Fig. 7.

### Example 5

### Preparation of the amorphous form of umeclidinium bromide

1-[2-(benzyloxy)ethyl]-4-(hydroxydiphenylmethyl)-1-azabicyclo[2.2.2]octane bromide (100 mg, 0.197 mmol, purity UPLC 98.89%) is dissolved at the temperature of 25°C in a water: *tert-*butanol mixture in the volume ratio of 6:4 (total 70 ml). The clear solution is freeze-dried (a bath with a mixture of dry ice and ethanol, -70°C) and lyophilized (vacuum: 1.8 Pa for 72 h). An amorphous form of umeclidinium bromide was obtained (100 mg). This amorphous form was confirmed with DSC and X-ray powder diffraction. The X-ray powder diffraction pattern is shown in Fig. 8 and the DSC record in Fig. 9.

## Claims

1. Form A of umeclidinium bromide, exhibiting the following characteristic reflections in the X-ray powder pattern, with the use of CuKa radiation: 5.4; 10.9; 19.6 and 23.8 ± 0.2° 2-theta.

2. Form A according to claim 1, exhibiting the following characteristic reflections in the X-ray powder pattern, with the use of CuKa radiation: 9.5; 16.3; 17.7; 21.6 and 27.5 ± 0.2° 2-theta.

3. Form A according to claims 1 or 2, having the melting point of 167.6°C as determined by means of DSC.

4. Form A according to claims 1 to 3, obtainable by crystallization from a mixture of an alcohol of the general formula ROH, where R is a C1-C4 carbon chain, and water, preferably from a methanol and water mixture.

5. Form A according to claim 4, wherein the crystallization mixture contains methanol and water in the volume ratio of 1:1 to 2:1.

6. Form B of umeclidinium bromide, exhibiting the following characteristic reflections in the X-ray powder pattern, with the use of CuKa radiation: 6.7; 18.3; 20.6 and 24.9 ± 0.2° 2-theta.

7. Form B according to claim 6, exhibiting the following characteristic reflections in the X-ray powder pattern, with the use of CuKa radiation: 14.6; 15.9; 19.2; 23.0 and 23.8 ± 0.2° 2-theta.

8. Form B according to claims 6 or 7, having the melting point of 232°C as determined by means of DSC.

9. Form B of umeclidinium bromide, which is obtained from form A as defined in any one of claims 1 to 5 at a temperature over 180°C.

10. Form B according to claims 6 to 8, obtainable from form A by drying at atmospheric pressure at a temperature of 200°C.

11. An amorphous form of umeclidinium bromide.

12. The amorphous form of umeclidinium bromide according to claim 11, exhibiting a characteristic amorphous halo in the X-ray powder pattern with the use of CuKa radiation.

13. The amorphous form according to claims 11 or 12, exhibiting the glass transition temperature of 81 °C as determined with the use of DSC.

14. The amorphous form according to claims 11 to 13, obtainable by dissolution of umeclidinium bromide in a water and *tert-*butanol mixture, freeze-drying and subsequent lyophilization.

15. The amorphous form according to claim 14, wherein the volume ratio of water and *tert-*butanol is 6:4.
